# FASCICULE DE BREVET EUROPEEN

(11) **EP 3 172 183 B1**
(45) Date de publication et mention de la délivrance du brevet: **30.09.2020**
(21) Numéro de dépôt: 15741179.4
(22) Date de dépôt: 22.07.2015
(51) Int. Cl.: C07C 29/76, C07C 31/20

(54) **PROCÉDÉ DE PURIFICATON DE GLYCOL UTILISÉ COMME AGENT ANTI-HYDRATE**
VERFAHREN ZUR REINIGUNG VON ALS HYDRATINHIBITOR VERWENDETEM GLYCOL
METHOD FOR PURIFYING GLYCOL USED AS A HYDRATE INHIBITOR

(30) Priorité: 25.07.2014 FR 1457249
(43) Date de publication de la demande: 31.05.2017
(73) Titulaire: Novasep Process, 54340 Pompey (FR)
(72) Inventeur: SCHAB, Frédéric, F-69007 Lyon (FR); COTILLON, Michel, F-95510 Vétheuil (FR); EBRAN, Teddy, F-38230 Pont de Chéruy (FR); GAVROY, Vincent, 69300 Caluire et Cuire (FR)
(74) Mandataire: Bandpay & Greuter
(86) Numéro de dépôt international: PCT/EP2015/066765
(87) Numéro de publication internationale: WO 2016/012500

(56) Documents cités:
- WO-A1-2014/031269
- WO-A1-2014/035740
- US-A- 2 771 193

## Description

### DOMAINE DE L'INVENTION

La présente invention concerne un procédé de purification de glycol, notamment dans le contexte de la régénération d'un agent anti-hydrate utilisé pour le transport des hydrocarbures.

### ARRIERE-PLAN TECHNIQUE

L'extraction d'hydrocarbures de gisements sous-marins s'effectue en récupérant un mélange polyphasique constitué d'hydrocarbures gazeux (notamment gaz naturel) et/ou liquides et d'eau de mer du gisement.

Dans certaines conditions de basses températures et de hautes pressions régnant au fond de la mer, le gaz naturel peut former avec l'eau un solide cristallin par liaisons de type Van der Waals, appelé hydrate de gaz ou plus simplement hydrate.

Les hydrates sont susceptibles d'obstruer les conduites d'hydrocarbures, ce qui peut provoquer l'arrêt complet de la production et l'endommagement des équipements de surface comme les vannes et les instruments de mesure.

Pour éviter la formation d'hydrates, il est connu d'utiliser des agents anti-hydrates, ou inhibiteurs d'hydrates, et plus particulièrement des glycols, tels que le monoéthylène glycol (MEG, le plus répandu), le diéthylène glycol (DEG) ou le triéthylène glycol (TEG). Le glycol est généralement injecté sous forme concentrée dans le mélange polyphasique extrait, au niveau du puits d'extraction. Il se dilue alors dans la phase aqueuse du mélange polyphasique et est mélangé avec des impuretés, telles que des sels minéraux issus de la mer ou de la roche. Les principaux ions présents dans le mélange polyphasique sont les ions sodium, potassium, magnésium, calcium, chlorures, sulfates et carbonates.

Le mélange polyphasique contenant les hydrocarbures gazeux et/ou liquides et l'eau chargée en sels et en glycol transite généralement par un réservoir situé au fond de la mer avant de remonter sous l'effet de la pression à la surface via une conduite pour être traité sur une plateforme.

Après séparation des hydrocarbures gazeux et liquides, l'eau chargée en sels et en glycol (appelée également « *glycol riche* ») doit être traitée pour pouvoir récupérer et recycler le glycol.

Une technique connue de traitement consiste à d'abord concentrer la solution par évaporation de l'eau et ensuite distiller le glycol, ces opérations, pouvant être effectuées dans une même colonne de distillation. Les sels cristallisent et se déposent au fond de la colonne de distillation avant d'être évacués vers une centrifugeuse qui permet de séparer le résidu de distillation des cristaux en pied de colonne. On récupère ainsi un flux de glycol purifié et concentré (également appelé « *lean glycol* », c'est-à-dire « *glycol pauvre* »), qui peut être recyclé. Cette technique présente plusieurs inconvénients.

La distillation de l'intégralité du flux de glycol représente une consommation d'énergie et des coûts opératoires très importants.

Les sels d'ions divalents tels que le carbonate ou le sulfate de calcium ou de magnésium ont une solubilité faible en phase aqueuse et forment un précipité sur les parois des tuyaux et des échangeurs thermiques, ce qui diminue leur capacité d'échange et par conséquent augmente la consommation énergétique. L'encrassement des installations peut en outre nécessiter des nettoyages fréquents. Ainsi les concentrations en ions divalents du glycol riche doivent être contrôlées, particulièrement lorsque la régénération de celui-ci implique des températures pouvant aller jusqu'à 150°C, car la solubilité des sels divalents diminue avec l'augmentation de la température.

Le document CA 2838617 présente un procédé de prétraitement de glycol riche par chauffage pour dégazer et précipiter les cations divalents associés aux carbonates avant la régénération.

Le document WO 2009/017971 propose un procédé de précipitation chimique des cations divalents par ajout de Na₂CO₃ et contrôle du pH alcalin avec de la soude, formant du CaCO₃ et du MgCO₃. La croissance des cristaux de CaCO₃ est un processus lent impliquant des temps de séjour importants.

Le document GB 2473213 propose un procédé similaire avec un maintien des conditions de température et de pH alcalin permettant de faire grossir les cristaux. Il propose en plus d'injecter un gaz inerte au fond de la cuve pour éliminer les impuretés par flottation : les impuretés s'adsorbent à la surface des bulles de gaz pour créer une mousse éliminée à la surface du glycol.

Le document US 2010/0191023 propose de pratiquer un ajout de cristaux de CaCO₃ dans du glycol riche pour accélérer la vitesse de croissance des cristaux. Les cristaux sont ensuite décantés et le surnageant est filtré, donnant un glycol pauvre avec une teneur résiduelle en CaCO₃ inférieure à 10 ppm.

Tous les procédés par précipitation des sels divalents décrits ci-dessus sont pratiqués sur le glycol riche avant l'évaporation de l'eau et présentent plusieurs inconvénients. D'une part, ils nécessitent un temps de contact important lié à la cristallisation et donc des cuves de cristallisation de gros volume. D'autre part ils nécessitent l'emploi de produits chimiques comme le Na₂CO₃ et le NaOH qu'il faut ensuite éliminer durant l'étape de distillation avec les sels monovalents non éliminés par l'étape de précipitation.

La séparation entre le glycol et les cristaux de sels peut être réalisée selon plusieurs techniques distinctes. Le document FR 2846323 décrit l'utilisation de la filtration, de la centrifugation ou de la séparation par ultrasons.

La séparation et l'évacuation des sels cristallisés suite à la distillation du glycol est une étape délicate à mettre en œuvre, avec des risques importants de colmatage des filtres et d'obturation des tuyaux. Dans le cas de la séparation centrifuge, les appareils rotatifs mis en œuvre requièrent des opérations de maintenance régulières.

Pour parer à ces difficultés liées à la présence de cristaux, le document US 2005/0022665 propose une purification glycol / sels par nanofiltration du glycol riche avant évaporation de l'eau, la membrane retenant une partie des glycols et laissant passer en partie les sels en solution dans le perméat. Mais d'une part, cette technique ne permet qu'une déminéralisation partielle du glycol riche, avec un taux de récupération insuffisant. D'autre part, c'est une technique coûteuse qui requiert une surface membranaire proportionnelle au flux traité et une consommation énergétique relativement élevée liée à la pression osmotique du glycol riche.

De la même façon, le document US 5,505,855 décrit la purification de TEG non aqueux présentant une viscosité importante par osmose inverse, le TEG étant retenu par la membrane qui laisse passer les sels.

Le document FR 2711650 enseigne l'utilisation d'un procédé d'électrodialyse. Le glycol riche est préalablement filtré pour éliminer les matières en suspension présentes. Le filtrat est ensuite traité par électrodialyse, qui consiste à déminéraliser le glycol par passage sélectif des anions et des cations à travers des membranes perméables uniquement aux uns ou aux autres grâce à l'action d'un courant électrique et d'électrodes disposées de part et d'autre de la pile de membranes. Le document enseigne également l'utilisation de résines échangeuses d'ions pour fixer sélectivement les anions ou les cations.

Mais l'électrodialyse a pour inconvénient de ne pas éliminer les sels dans une proportion suffisante, si bien qu'elle doit en pratique être complétée par une étape dite de finition capable d'éliminer les sels résiduels ; de plus elle offre un taux de récupération du glycol qui est inférieur à 97 %. Et d'un autre côté, l'utilisation de la technique d'échange d'ions a pour inconvénient de nécessiter une régénération fréquente et coûteuse de la résine. Cette régénération suppose en effet l'utilisation de quantités importantes d'acide et de base. Par ailleurs, le volume total d'adsorbant mis en œuvre, ainsi que le volume total de produits chimiques utilisés pour sa régénération, sont directement proportionnels à la quantité de sels à éliminer et donc à la charge du glycol riche à traiter. Un autre inconvénient réside dans les volumes importants d'effluents acides et basiques à traiter et à éliminer.

Le document FR 2899822 est un autre exemple de technique de déminéralisation d'agent anti-hydrate par échange d'ions avec un support.

En dehors du domaine de la régénération des agents anti-hydrates, le document WO 94/26684 revendique un procédé de purification et de récupération d'un mélange de DEG et d'oligomères de téréphtalate pour la production de polyéthylène téréphtalate, contenant des oxydes métalliques utilisés comme catalyseurs, des colorants et d'autres impuretés à éliminer. Le mélange est chauffé pour permettre aux oligomères d'être solubilisés dans le DEG, puis il passe successivement par les étapes suivantes : filtrage sur charbon actif, passage sur une résine cationique, passage sur une résine anionique, lit mélangé et distillation. L'utilisation d'une étape d'échange d'ions comporte les inconvénients mentionnés ci-dessus.

Le document GB 2091580 propose une séparation chromatographique pour purifier et recycler de l'éthylène glycol contenu dans les eaux de lavage issues de la production d'oxyde d'éthylène. Le document WO 2014/035740 concerne un procédé de réduction des contaminants dans un produit glycolique obtenu par hydrogénolyse de saccharides issus de sources biologiques ou renouvelables, par chromatographie d'exclusion d'ions suivie d'une distillation.

Dans ces deux documents, le but de la purification chromatographique est de séparer le glycol d'acides organiques qui forment des sels avec l'ion sodium.

Le document US 2,771,193 décrivait d'ailleurs déjà l'utilisation de la technique de chromatographie d'exclusion d'ions pour séparer un glycol du chlorure de sodium.

Ces deux documents ne suggèrent pas la possibilité d'utiliser cette technique pour séparer un glycol d'un mélange complexe d'ions.

Le document WO 2014/031269 décrit un procédé pour éliminer les cations divalents d'un flux de monoéthylène glycol à l'aide d'une résine échangeuse de cations.

Il existe donc encore un besoin de simplifier et d'améliorer les procédés de régénération des agents anti-hydrates à base de glycol, et notamment un besoin de fournir des solutions efficaces et économiques en remplacement de la distillation du flux de glycol riche et de la cristallisation des sels.

Il existe donc un besoin pour un procédé de régénération d'agent anti-hydrate qui soit robuste, fiable, productif, peu consommateur d'énergie, peu ou pas consommateur de produits chimiques, et générant un minimum d'effluents faciles à recycler.

### RESUME DE L'INVENTION

L'invention concerne en premier lieu un procédé de purification comprenant :
- la fourniture d'un flux comprenant un glycol, des ions monovalents et des ions multivalents ;
- le traitement de ce flux par chromatographie d'exclusion d'ions comprenant :
   ▪ l'injection du flux dans une unité chromatographique comprenant une phase stationnaire échangeuse d'ions ;
   ▪ l'injection d'un éluant dans l'unité chromatographique ;
   ▪ la collecte d'une fraction en sortie de l'unité chromatographique ;
la fraction collectée étant enrichie en glycol et appauvrie en ions monovalents et en ions multivalents par rapport au flux.

Selon un mode de réalisation, le procédé comprend une étape ultérieure de concentration de la fraction collectée.

Selon un mode de réalisation, le glycol est choisi parmi le monoéthylène glycol, le diéthylène glycol, le triéthylène glycol et les mélanges de ceux-ci.

Selon un mode de réalisation, les ions monovalents comprennent des cations monovalents, et les ions multivalents comprennent des cations divalents ; et, de préférence, les cations monovalents comprennent des cations Na⁺ et/ou K+, et les cations divalents comprennent des cations Ca²⁺ et/ou Mg²⁺.

Selon un mode de réalisation, le flux présente un rapport en équivalents de cations divalents sur les cations totaux de 5 à 90 %, préférablement de 8 à 75 % et de manière préférée de 10 à 50 %.

Selon un mode de réalisation, l'unité chromatographique est une unité multi-colonnes à lit non-statique, et de préférence une unité chromatographique à lit mobile simulé, ou à lit mobile réel, ou à lit mobile simulé amélioré, ou à lit mobile simulé séquentiel, et de manière particulièrement préférée à lit mobile simulé séquentiel.

Selon un mode de réalisation, l'éluant est de l'eau, de préférence de l'eau déminéralisée ou de l'eau issue de l'étape de concentration de la fraction collectée.

Selon un mode de réalisation, le traitement du flux par chromatographie d'exclusion d'ions est effectué à une température de 10 à 160°C, de préférence de 40 à 100°C, et plus particulièrement de 55 à 90°C.

Selon un mode de réalisation, la phase stationnaire est une résine cationique qui est en partie sous forme cationique monovalente et en partie sous forme cationique divalente, la forme cationique monovalente et la forme cationique divalente correspondant de préférence à des ions présents dans le flux.

Selon un mode de réalisation, dans la fraction collectée, au moins 50 mol.%, de préférence au moins 80 mol.%, encore de préférence au moins 85 mol.%, de manière plus particulièrement préférée au moins 90 mol.% et de manière encore plus préférée au moins 95 mol.% des ions monovalents et des ions multivalents présents dans le flux sont éliminés.

Selon un mode de réalisation, au moins 20 kg de glycol, de préférence au moins 40 kg de glycol, de manière plus particulièrement préférée au moins 90 kg de glycol par litre de phase stationnaire passe dans l'unité chromatographique avant que la phase stationnaire subisse un lavage ; et, idéalement, la phase stationnaire ne subit aucun lavage.

L'invention concerne également un procédé de régénération d'un agent anti-hydrate, comprenant :
- la fourniture d'un mélange multiphasique comprenant des hydrocarbures, un glycol et des sels dissous ;
- la séparation des hydrocarbures de ce mélange, permettant de récupérer une phase aqueuse ;
- l'application du procédé de purification tel que décrit ci-dessus à la phase aqueuse en tant que flux, et la récupération d'une composition d'agent anti-hydrate régénérée sous la forme de la fraction collectée, et le cas échéant concentrée.

Selon un mode de réalisation, le procédé comprend une étape de clarification de la phase aqueuse avant l'application du procédé de purification, de préférence par filtration et/ou une étape de concentration de la phase aqueuse.

L'invention concerne également une installation de purification comprenant :
- une ligne d'amenée d'un flux comprenant un glycol, des ions monovalents et des ions multivalents ;
- une ligne d'amenée d'éluant ;
- une unité chromatographique comprenant une phase stationnaire échangeuse d'ions, alimentée par la ligne d'amenée du flux et par la ligne d'amenée d'éluant et configurée pour réaliser une chromatographie d'exclusion d'ions, et dans laquelle la phase stationnaire est une résine cationique qui est en partie sous forme cationique monovalente et en partie sous forme cationique divalente, la forme cationique monovalente et la forme cationique divalente correspondant à des ions présents dans le flux ;
- une ligne de collecte de fraction enrichie en glycol et appauvrie en ions monovalents et en ions multivalents par rapport au flux, connectée en sortie de l'unité chromatographique.

Selon un mode de réalisation, la ligne de collecte de fraction enrichie en glycol et appauvrie en ions monovalents et en ions multivalents alimente une unité de concentration de la fraction enrichie en glycol et appauvrie en ions monovalents et en ions multivalents.

Selon un mode de réalisation, le glycol est choisi parmi le monoéthylène glycol, le diéthylène glycol, le triéthylène glycol et les combinaisons de ceux-ci.

Selon un mode de réalisation, les ions monovalents comprennent des cations monovalents, et les ions multivalents comprennent des cations divalents ; et de préférence, les cations monovalents comprennent des cations Na⁺ et/ou K+, et les cations divalents comprennent des cations Ca²⁺ et/ou Mg²⁺.

Selon un mode de réalisation, le flux présente un rapport en équivalents de cations divalents sur les cations totaux de 5 à 90 %, ou de 8 à 75 %, ou de 10 à 50 %.

Selon un mode de réalisation, l'unité chromatographique est une unité multi-colonnes à lit non-statique, et de préférence une unité chromatographique à lit mobile simulé, ou à lit mobile réel, ou à lit mobile simulé amélioré, ou à lit mobile simulé séquentiel, et de manière particulièrement préférée à lit mobile simulé séquentiel.

Selon un mode de réalisation, l'éluant est de l'eau, de préférence de l'eau déminéralisée ; et, de manière particulièrement préférée, une ligne de recyclage d'eau est prévue entre l'unité de concentration de la fraction enrichie en glycol et appauvrie en ions monovalents et en ions multivalents et la ligne d'amenée d'éluant.

Selon un mode de réalisation, l'installation est pourvue de moyens de régulation de température configurés pour que l'unité chromatographique opère à une température de 10 à 160°C, de préférence de 40 à 100°C, et plus particulièrement de 55 à 90°C. La phase stationnaire est une résine cationique qui est en partie sous forme cationique monovalente et en partie sous forme cationique divalente, la forme cationique monovalente et la forme cationique divalente correspondant à des ions présents dans le flux.

L'invention concerne également une installation de régénération d'un agent-hydrate, comprenant :
- une ligne d'amenée d'un mélange multiphasique comprenant des hydrocarbures, un glycol et des sels dissous ;
- une unité de séparation des hydrocarbures alimentée par cette conduite d'amenée de mélange polyphasique ;
- une ligne de prélèvement de phase aqueuse issue de l'unité de séparation des hydrocarbures ;
- l'installation de purification décrite ci-dessus, dans laquelle la ligne d'amenée de flux est alimentée par la ligne de prélèvement de phase aqueuse ;
- une ligne de collecte de composition d'agent anti-hydrate régénérée, alimentée par la ligne de collecte de fraction enrichie en glycol et appauvrie en ions monovalents et en ions multivalents ou le cas échéant par une ligne de collecte issue de l'unité de concentration de la fraction enrichie en glycol et appauvrie en ions monovalents et en ions multivalents.

Selon un mode de réalisation, cette installation comprend une unité de clarification de la phase aqueuse, de préférence une unité de filtration, et/ou une unité de concentration de la phase aqueuse, en amont de l'installation de purification.

La présente invention permet de surmonter les inconvénients de l'état de la technique. Elle fournit plus particulièrement un procédé de purification de glycol qui peut être appliqué notamment dans un procédé de régénération d'agent anti-hydrate à base de glycol. Ce procédé est simplifié et amélioré, plus efficace et plus économique que les procédés de l'état de la technique. Le procédé selon l'invention est robuste, fiable, productif, peu consommateur d'énergie, peu ou pas consommateur de produits chimiques, et il génère un minimum d'effluents faciles à recycler.

Ce procédé repose sur une étape de séparation chromatographique qui ne nécessite pas (ou quasiment pas) de lavage ou de reconditionnement de la phase stationnaire.

Cela est accompli grâce à la découverte que la technique de la chromatographie d'exclusion d'ions permet de séparer de manière très efficace le glycol à la fois des ions monovalents et des ions multivalents (notamment divalents) généralement présents dans le mélange polyphasique qui est collecté lors de l'extraction d'hydrocarbures. Cette séparation est effectuée en une seule étape de chromatographie (c'est-à-dire qu'une seule phase stationnaire est utilisée, répartie dans une ou plusieurs colonnes, et un seul éluant).

Compte tenu de la différence d'affinité des résines échangeuses d'ions pour les ions monovalents et les ions multivalents, on pouvait s'attendre à obtenir une performance médiocre de séparation, du fait de l'apparition attendue de formes ioniques différentes selon les régions de la résine et de fronts de déplacement d'ions. De plus, les ions multivalents (tels que les ions Ca²⁺ notamment) sont généralement de taille plus importante que les ions monovalents (Na⁺, H⁺...), si bien que leur présence réduit le volume interne de la phase stationnaire, ce qui est susceptible d'avoir un impact négatif sur la qualité de la séparation. C'est ce que l'on observe dans le cadre de la purification des sucres par exemple.

Or il a été constaté de manière surprenante qu'une excellente séparation entre le glycol et les sels dissous est obtenue de manière stable dans le temps. On pouvait également s'attendre à l'apparition de phénomènes de précipitation, notamment lorsque des ions calcium sont présents. Mais il a été constaté de manière surprenante que ce n'est pas le cas, et à nouveau qu'une excellente séparation entre le glycol et les sels est obtenue de manière stable dans le temps.

### BREVE DESCRIPTION DES FIGURES

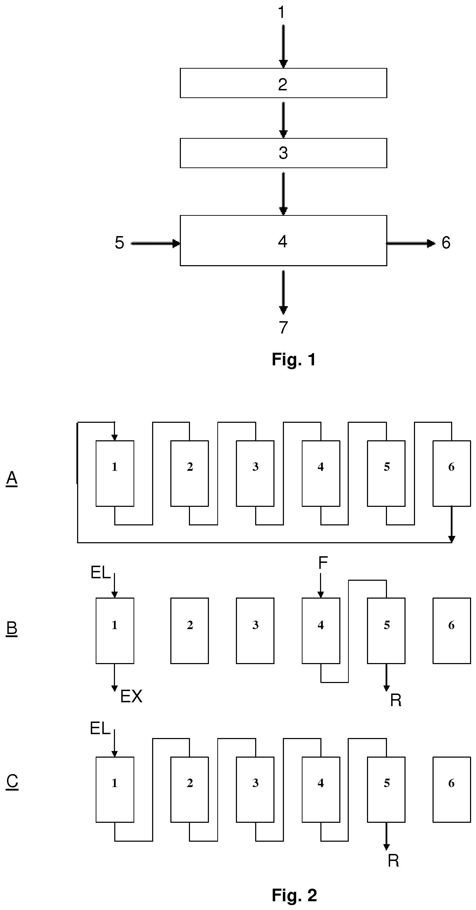

La **figure 1** est un schéma-bloc illustrant le principe de l'invention selon un mode de réalisation particulier.
La **figure 2** représente de manière schématique une unité chromatographique permettant de mettre en œuvre le procédé selon un mode de réalisation de l'invention.

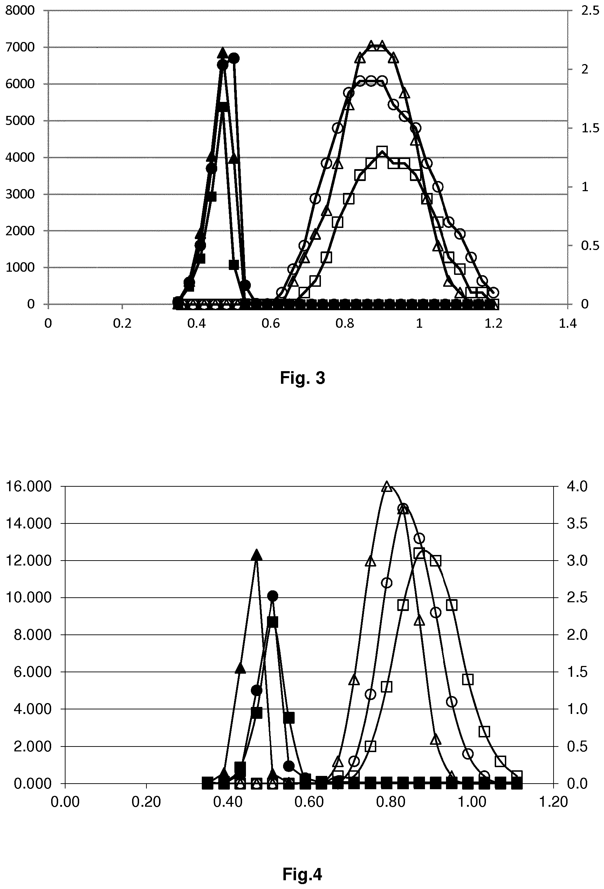

La **figure 3** est un graphique représentant les chromatogrammes obtenus dans l'exemple 1, par injection de trois solutions respectivement de MEG, DEG et TEG contenant les mêmes concentrations en glycols et en sels. En abscisse figure le volume d'éluant injecté (en unités BV, signifiant volume de résine). Sur l'axe des ordonnées de gauche figure la conductivité de la fraction concernée en µS/cm, et sur l'axe des ordonnées de droite figure la teneur en matières sèches en % Brix. La courbe avec les symboles triangles est celle obtenue avec la solution de MEG ; la courbe avec les symboles carrés est celle obtenue avec la solution de DEG ; et la courbe avec les symboles ronds est celle obtenue avec la solution de TEG. Les symboles coloriés en noir correspondent à la mesure de conductivité, et les symboles non coloriés correspondent à la mesure de teneur en matières sèches.
La **figure 4** est un graphique représentant les chromatogrammes obtenus dans l'exemple 2, par injection d'une solution industrielle de MEG riche sur une résine cationique sous trois formes ioniques distinctes : K, Ca et 65 % Ca - 35 % K. En abscisse figure le volume d'éluant injecté (en unités BV). Sur l'axe des ordonnées de gauche figure la conductivité de la fraction concernée en mS/cm, et sur l'axe des ordonnées de droite figure la teneur en matières sèches en % Brix. La courbe avec les symboles triangles est celle obtenue avec la résine sous forme K ; la courbe avec les symboles carrés est celle obtenue avec la résine sous forme Ca ; et la courbe avec les symboles ronds est celle obtenue avec la résine sous forme 65 % Ca - 35 % K. Les symboles coloriés en noir correspondent à la mesure de conductivité, et les symboles non coloriés correspondent à la mesure de teneur en matières sèches.

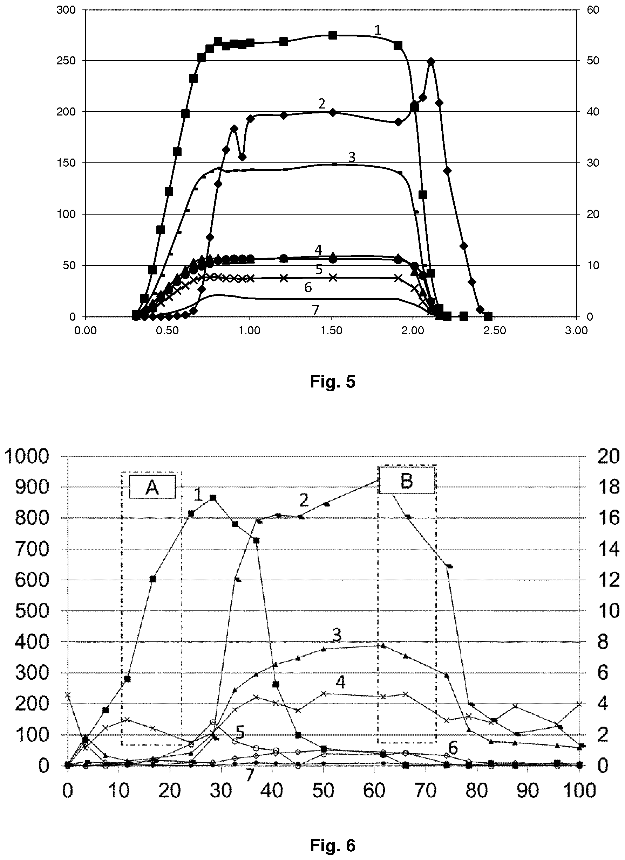

La **figure 5** est un graphique représentant les chromatogrammes obtenus dans l'exemple 3, par surcharge avec une solution industrielle de MEG riche présentant une concentration en sels supérieure à sa concentration en MEG, sur une résine cationique forte résistante aux variations de pression osmotique, préalablement équilibrée ioniquement avec ce produit, puis rincée à l'eau déminéralisée. En abscisse figure le volume injecté (en unités BV). En ordonnée figure la concentration de chaque espèce en g/L. L'échelle de droite est utilisée pour les ions potassium et magnésium, et l'échelle de gauche est utilisée pour les autres espèces. La courbe 1 correspond à la concentration totale en sels, la courbe 2 correspond à la concentration en MEG, la courbe 3 correspond à la concentration en Cl⁻, la courbe 4 correspond à la concentration en Na⁺, la courbe 5 correspond à la concentration en Ca²⁺, la courbe 6 correspond à la concentration en K⁺ et la courbe 7 correspond à la concentration en Mg²⁺.
La **figure 6** est un graphique représentant les chromatogrammes obtenus dans l'exemple 4, avec une séparation dans un système chromatographique continu de type SSMB. En abscisse figure le pourcentage du temps de cycle. En ordonnée figure la concentration des différentes espèces en g/L. L'échelle de droite est utilisée pour tous les sels, et l'échelle de gauche est utilisée pour le MEG. La courbe 1 correspond à la concentration en MEG, la courbe 2 correspond à la concentration en Cl⁻, la courbe 3 correspond à la concentration en Na⁺, la courbe 4 correspond à la concentration en Ca²⁺, la courbe 5 correspond à la concentration en acétate, la courbe 6 correspond à la concentration en K⁺ et la courbe 7 correspond à la concentration en Mg²⁺. La partie du graphique marquée A correspond à l'extrait, et la partie marquée B correspond au raffinat.

### DESCRIPTION DE MODES DE REALISATION DE L'INVENTION

L'invention est maintenant décrite plus en détail et de façon non limitative dans la description qui suit.

L'invention concerne la séparation de glycol en solution aqueuse vis-à-vis de sels dissous sous forme d'ions monovalents et multivalents. Dans ce qui suit, l'invention est plus particulièrement décrite dans une application de ce principe pour la régénération d'un agent anti-hydrate.

Par « *agent anti-hydrate* » on entend un produit susceptible d'inhiber, empêcher, prévenir ou réduire la formation d'hydrates de gaz dans des mélanges polyphasiques comprenant de l'eau et des hydrocarbures liquides et/ou gazeux.

Dans le cadre de l'invention, l'agent anti-hydrate est une composition comprenant un glycol. Le glycol peut être de manière non-limitative du MEG, du DEG ou du TEG. On peut également utiliser une combinaison de plusieurs de ces composés.

Par « *régénération* », on entend la purification de l'agent anti-hydrate vis-à-vis de contaminants indésirables, et notamment de sels minéraux.

L'agent anti-hydrate usé est d'abord récupéré sous forme d'un mélange polyphasique, qui est en général un mélange d'hydrocarbures gazeux et / ou liquides, tel qu'un mélange d'hydrocarbures extrait issu d'un gisement, mélangé notamment avec de l'eau, de l'agent anti-hydrate (glycol) et des contaminants tels que des sels minéraux.

On sépare dans un premier temps une phase aqueuse (contenant le glycol) d'une phase gazeuse et d'une phase huileuse. Pour ce faire, on peut par exemple faire passer le mélange dans une première colonne de séparation ou compartiment de détente qui permet de séparer la phase gazeuse des phases liquides. Puis, on fait passer les phases liquides dans un deuxième compartiment, où la phase huileuse et la phase aqueuse sont séparées selon leurs densités, par exemple par décantation.

Le gaz et les hydrocarbures liquides sont évacués et stockés vers des réservoirs. La phase aqueuse subit ensuite les traitements suivants prévus par l'invention.

En faisant référence à la **figure 1**, selon un mode de réalisation de l'invention, la phase aqueuse 1 peut subir d'abord une étape d'ajustement de la température 2 au moyen d'un échangeur de chaleur - et ce afin que la température de la phase aqueuse soit adaptée en fonction des paramètres de la séparation chromatographique ultérieure. Généralement il s'agit d'une étape de chauffage (mais un refroidissement peut être approprié dans certains cas).

Ensuite la phase aqueuse peut subir une étape de clarification 3 (dans une unité de clarification). Cette étape peut également être prévue avant l'étape d'ajustement de la température. Chacune des deux étapes est optionnelle. On peut ainsi avoir un ajustement de la température seulement, ou une clarification seulement, ou un ajustement de la température puis une clarification comme illustré, ou bien encore une clarification puis un ajustement de la température.

L'étape de clarification 3 consiste à réduire ou éliminer les matières en suspension. On peut utiliser dans ce but par exemple une filtration au moyen d'un filtre à poche ou bobine, filtre-panier ou filtre-presse, une centrifugation, une filtration sur terre avec ou sans pré-couche et/ou nourrissage, une filtration frontale ou encore une filtration tangentielle sur membrane de type microfiltration ou ultrafiltration, ou encore une filtration sur diatomées ou sur membrane céramique.

Ensuite, on met en œuvre l'étape de chromatographie 4 qui constitue le cœur de l'invention. Dans ce qui suit, on désigne par « *flux en entrée* » le flux aqueux qui est soumis à l'étape de chromatographie 4.

En sortie de cette étape de chromatographie 4, on collecte une fraction 6 enrichie en glycol et appauvrie en ions, et une fraction 7 enrichie en ions et appauvrie en glycol. L'étape de chromatographie 4 est mise en œuvre en utilisant par ailleurs un flux d'éluant 5 (par exemple un flux d'eau déminéralisée).

Par « *fraction enrichie en glycol et appauvrie en ions* », on entend une fraction dans laquelle le rapport de concentrations molaires glycol / ions totaux est supérieur à celui du flux en entrée de la chromatographie (indépendamment des effets de concentration ou de dilution globale vis-à-vis de l'eau). De préférence, ce rapport est supérieur d'un facteur au moins égal à 2, ou au moins égal à 3, ou au moins égal à 5, ou au moins égal à 8, ou au moins égal à 10. Cette fraction est également appelée « extrait ».

En général, ladite fraction enrichie en glycol est moins concentrée en glycol que le flux en entrée. Le taux de dilution (rapport de la concentration en glycol en g/L dans la fraction enrichie en glycol sur la concentration en glycol en g/L dans le flux en entrée) peut être de préférence de 0,4 à 1,00, notamment de 0,60 à 0,99, et plus particulièrement de 0,65 à 0,95.

La fraction enrichie en glycol (et appauvrie en ions) peut ensuite subir une étape optionnelle de concentration (visant à réduire la teneur en eau et donc augmenter la teneur en glycol), par exemple dans un évaporateur. Cette étape ultérieure de concentration est particulièrement appropriée pour des flux très chargés en sels, pour lesquels il importe de procéder à une déminéralisation avant tout passage dans un évaporateur. Alternativement, on peut prévoir une étape de concentration avant l'étape de chromatographie 4, ce qui peut permettre de diminuer la taille de l'unité chromatographique (qui sera décrite plus en détail ci-dessous). On peut également prévoir une concentration avant et après l'étape de chromatographie 4. Dans ce cas, les deux étapes de concentration peuvent être mises en œuvre sur un même équipement. On peut dans ce cas disposer l'unité chromatographique sur une ligne de recirculation d'un évaporateur.

Par « *fraction enrichie en ions et appauvrie en glycol* », on entend au contraire une fraction dans laquelle le rapport de concentrations molaires glycol / ions totaux est inférieur à celui du flux en entrée de la chromatographie (indépendamment des effets de concentration ou de dilution globale vis-à-vis de l'eau). De préférence, ce rapport est inférieur d'un facteur au moins égal à 2, ou au moins égal à 3, ou au moins égal à 5, ou au moins égal à 8, ou au moins égal à 10. Cette fraction est également appelée « *raffinat* »*.*

Le flux en entrée traité par chromatographie est une solution aqueuse de glycol avec en outre des ions monovalents et multivalents, notamment divalents, en solution. La composition de ce flux dépend fortement de la provenance du mélange polyphasique récolté. En particulier, la nature et la teneur des ions en solution dépend du lieu et de l'âge géologique du puits et de la nature des roches du gisement.

Toutefois, l'invention est robuste et permet de s'adapter à des compositions variées et de les traiter efficacement.

La teneur en glycol du flux en entrée peut varier de 1 à 1283 g/L et notamment de 200 à 900 g/L.

La concentration totale en sels du flux en entrée peut aller de 0,1 g/L jusqu'à la concentration limite de solubilité des différentes espèces présentes dans la solution.

Selon un mode de réalisation, le flux en entrée contient des cations Na⁺ et / ou K⁺ d'une part ; et des cations Ca²⁺ et / ou Mg²⁺ d'autre part.

Selon un mode de réalisation, le flux en entrée contient à la fois des cations Na⁺, K⁺, Ca²⁺ et Mg²⁺.

Il peut également contenir des anions tels que Cl⁻, SO₄²⁻ et CO₃²⁻, et plus particulièrement des anions Cl⁻, ceux-ci étant les plus solubles.

Il faut noter que de nombreux autres ions métalliques en solution peuvent être présents, tels que les ions Fe²⁺, Fe³⁺, Cu²⁺, Zn²⁺, etc. et ce même si des agents anticorrosion sont utilisés.

Plus particulièrement, le flux en entrée contient de préférence de 0,01 à 154 g/L d'ions Na⁺, de manière plus particulièrement préférée de 0,1 à 100 g/L et de manière encore plus particulièrement préférée de 0,5 à 80 g/L, de manière encore plus préférée de 1 à 40 g/L et notamment de 2 à 15 g/L.

Plus particulièrement, le flux en entrée contient de préférence de 0,01 à 178 g/L d'ions K⁺, de manière plus particulièrement préférée de 0,1 à 100 g/L et de manière encore plus particulièrement préférée de 0,2 à 50 g/L, de manière encore plus préférée de 0,3 à 10 g/L et notamment de 0,5 à 4 g/L.

Plus particulièrement, le flux en entrée contient de préférence de 0,01 à 268 g/L d'ions Ca²⁺, de manière plus particulièrement préférée de 0,1 à 200 g/L et de manière encore plus particulièrement préférée de 0,2 à 100 g/L, de manière encore plus préférée de 0,5 à 50 g/L et notamment de 1 à 10 g/L.

Plus particulièrement, le flux en entrée contient de préférence de 0,01 à 137 g/L d'ions Mg²⁺, de manière plus particulièrement préférée de 0,02 à 80 g/L et de manière encore plus particulièrement préférée de 1 à 30 g/L, de manière encore plus préférée de 0,05 à 10 g/L et notamment de 0,1 à 1 g/L.

On définit le rapport des cations divalents sur les cations totaux, en équivalents, comme le rapport des concentrations molaires totales des espèces concernées, pondérées par leur charge. Par exemple, une mole d'ions Ca²⁺ est équivalente à deux moles d'ions Na⁺.

Selon des modes de réalisation, ce rapport vaut de 5 à 10 % ; ou de 10 à 15 % ; ou de 15 à 20 % ; ou de 20 à 25 % ; ou de 25 à 30 % ; ou de 30 à 35 % ; ou de 35 à 40 % ; ou de 40 à 45 % ; ou de 45 à 50 % ; ou de 50 à 55 % ; ou de 55 à 60 % ; ou de 60 à 65 % ; ou de 65 à 70 % ; ou de 70 à 75 % ; ou de 75 à 80 % ; ou de 80 à 85 % ; ou de 85 à 90 % ; ou de 90 à 95 %.

Dans des modes de réalisation préférés, ce rapport vaut de 5 à 90 %, ou de 8 à 75 %, ou de 10 à 50 %.

L'étape de chromatographie 4 est une étape de chromatographie d'exclusion d'ions, qui est mise en œuvre dans une unité chromatographique appropriée qui comprend une phase stationnaire échangeuse d'ions, en général une résine échangeuse d'ions - celle-ci n'étant toutefois pas utilisée en mode d'échange d'ions.

Le principe de la chromatographie d'exclusion d'ions est que la phase stationnaire est saturée avec les mêmes ions que ceux qui sont présents dans le flux en entrée et que l'on cherche à réduire ou éliminer.

Dans la chromatographie d'exclusion d'ions, les ions présents dans le flux en entrée ont tendance à être très peu retenus par la phase stationnaire et à être élués plus rapidement que les espèces non-ioniques telles que le glycol.

La chromatographie d'exclusion d'ions est ainsi effectuée sans échange net d'ions sur la phase stationnaire au cours de la séparation.

La chromatographie d'exclusion d'ions peut être mise en œuvre simplement :
- en injectant le flux en entrée dans l'unité chromatographique ;
- en injectant un éluant dans l'unité chromatographique ;
- et en collectant les fractions susmentionnées en sortie de l'unité chromatographique.

La phase stationnaire utilisée est avantageusement une résine cationique forte ou faible, ou encore une résine anionique forte ou faible.

De préférence c'est une résine cationique forte.

La résine est avantageusement un copolymère de polystyrène et de divinylbenzène (DVB).

Selon un mode de réalisation la résine présente un taux de réticulation compris entre 2 et 12 % de DVB, de préférence entre 3 et 10 %, plus particulièrement entre 4 et 9 %, et de préférence entre 5 et 8 %.

La résine peut avoir par exemple une granulométrie (diamètre volumique moyen de particules Dv50) comprise entre 100 et 800 µm, de préférence entre 200 et 350 µm.

Il est avantageux de mettre en œuvre l'étape de séparation chromatographique à une température de 10 à 160°C, de préférence de 40 à 100°C, et plus particulièrement de 55 à 90°C. Des moyens de refroidissement ou de chauffage (avantageusement, de chauffage) sont prévus à cet effet.

A titre d'éluant, on utilise avantageusement de l'eau ou une solution aqueuse. De préférence on utilise de l'eau déminéralisée (et dégazée).

Le rapport volumique de la quantité d'éluant sur la quantité de flux en entrée traité est avantageusement inférieur ou égal à 3, de préférence inférieur ou égal à 2, de préférence inférieur ou égal à 0,9.

La phase stationnaire utilisée peut par exemple se trouver initialement sous la forme d'un cation divalent (notamment calcium ou magnésium), ou de manière plus préférée sous la forme d'un cation monovalent (notamment potassium ou très préférablement sodium).

Toutefois, en cours d'utilisation, la forme ionique de la phase stationnaire évolue pour atteindre un équilibre ionique avec le flux en entrée. Du fait de l'affinité différente des ions monovalents et multivalents pour la phase stationnaire, les proportions d'ions monovalents et multivalents sur la phase stationnaire sont toutefois généralement différentes de celles du flux en entrée.

Dans des modes de réalisation particuliers, la phase stationnaire est ainsi chargée avec de 5 à 95 %, de préférence de 20 à 90 % et de manière plus particulièrement préférée de 55 à 80 % avec des ions multivalents (et plus particulièrement des ions divalents) par rapport à la charge totale, ces proportions étant données en équivalents molaires.

Cette forme ionique de la phase stationnaire peut être mesurée par déplacement des ions fixés sur la phase stationnaire, par exemple par injection d'un excès d'un acide (tel que l'acide chlorhydrique), puis mesure des teneurs en ions élués, par exemple par chromatographie HPLC ionique.

L'étape de séparation chromatographique peut être discontinue (« *batch* »), semi-continue ou continue ; de préférence elle est semi-continue ou continue.

L'unité chromatographique peut être constituée d'une seule colonne. Mais de préférence, il s'agit d'une unité multi-colonnes, qui de préférence comporte 11 colonnes au plus, ou 8 colonnes au plus, encore de préférence 6 colonnes au plus et particulièrement 4 colonnes au plus, et idéalement moins de 4 colonnes.

L'unité chromatographique peut être à lit statique, ou de préférence à lit non-statique.

Une unité chromatographique à lit non-statique est un système multi-colonnes, dans lequel les positions relatives du lit de phase stationnaire et des points d'injection ou de collecte des flux se déplacent au cours du temps.

Des exemples de telles unités chromatographiques à lit non-statique sont les systèmes SMB (lit mobile simulé), iSMB (lit mobile simulé amélioré), SSMB (lit mobile simulé séquentiel), AMB (lit mobile réel), VARICOL™, MODICON™, POWERFEED™, MCSGP ou GSSR (chromatographie de gradient multi-colonnes).

Un système SMB comprend une pluralité de colonnes individuelles contenant un adsorbant, qui sont connectées en série. Le flux d'éluant traverse les colonnes selon une première direction. Les points d'injection du flux d'alimentation (flux à traiter) et de l'éluant, ainsi que les points de collecte des fractions séparées, sont décalés périodiquement et simultanément au moyen d'un ensemble de vannes. L'effet global est de simuler le fonctionnement d'une colonne unique contenant un lit mobile d'adsorbant solide, l'adsorbant solide se déplaçant dans une direction à contre-courant du flux d'éluant. Ainsi, un système SMB est composé de colonnes qui contiennent des lits stationnaires d'adsorbant solide à travers lesquels passe l'éluant, mais le fonctionnement est tel qu'un lit mobile continu à contre-courant est simulé.

La forme la plus conventionnelle d'un système SMB est le système SMB à quatre zones. D'autres formes possibles sont les systèmes SMB à trois zones et les systèmes SMB à deux zones (tels que décrits dans l'article « Two Section Simulated Moving Bed Process » de Kwangnam Lee, dans Separation Science and Technology 35(4):519-534, 2000, auquel il est fait expressément référence).

Dans les systèmes iSMB et SSMB, il y a au moins une étape dans laquelle le système fonctionne en boucle fermée, sans entrée ou sortie de produit.

Un système iSMB est tel que décrit dans les documents EP 0342629 et US 5,064,539, auxquels il est fait expressément référence.

Un système SSMB découpe les introductions et collectes des flux en sous séquences appliquées de façons périodiques.

D'autres variantes des systèmes SMB sont : le système SMB variant dans le temps et le système POWERFEED™, tels que décrits dans le document US 5,102,553 et dans l'article « PowerFeed opération of simulated moving bed units: changing flow-rates during the switching interval », de Zhang et al. dans Journal of Chromatography A, 1006:87-99, 2003, auxquels il est fait expressément référence; le système MODICON™, tel que décrit dans le document US 7,479,228, auquel il est fait expressément référence ; et le système SMB avec recirculation interne, tel que décrit dans le document US 8,282,831, auquel il est fait expressément référence.

Un système AMB présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

Un système de chromatographie VARICOL™ est tel que décrit dans les documents US 6,136,198, US 6,375,839 US 6,413,419 et US 6,712,973, auxquels il est fait expressément référence. Un système VARICOL™ comprend une pluralité de colonnes individuelles contenant un adsorbant qui sont reliées en série. On fait passer l'éluant dans les colonnes selon une première direction. Contrairement au système SMB, les points d'injection pour le flux à séparer et pour l'éluant et les points de collecte des fractions séparées dans le système sont déplacés périodiquement mais de manière asynchrone, au moyen d'un ensemble de vannes. L'effet global est de créer des zones de séparation de longueur variable dans le temps, allouant ainsi la phase stationnaire de manière dynamique dans les zones où elle est la plus utile, et permettant une puissance de séparation similaire avec moins d'unités de séparation chromatographique et une productivité accrue. Contrairement à un système SMB, un système VARICOL™ ne simule pas le fonctionnement d'une colonne unique contenant un lit mobile d'adsorbant solide, l'adsorbant solide se déplaçant dans une direction à contre-courant du flux d'éluant, et ainsi le principe de fonctionnement du VARICOL™ ne peut pas être mis en œuvre dans un système AMB équivalent.

Un exemple préféré de lit mobile simulé est représenté en référence à la **figure 2**, sous la forme d'un système de chromatographie de type lit mobile simulé séquentiel (SSMB) sur six cellules ou colonnes. Ce système peut être opéré selon un fonctionnement cyclique en quatre phases.
- Phase n°1 (partie A de la figure) : phase de boucle, durant laquelle on maintient une circulation continue en boucle fermée sur toutes les cellules placées en série, pour déplacer le volume interstitiel d'une cellule vers la suivante, sans injection d'éluant.
- Phase n°2 (partie B de la figure) : charge / injection de charge. La charge du flux (F) est injectée en tête de la quatrième cellule. Simultanément, on collecte un volume sensiblement identique de raffinat (R) en sortie de la cinquième cellule. Les cellules 4 et 5 constituent ici la zone 3. Les cellules 2 et 3 constituent la zone de séparation entre extrait et injection de charge. Elles constituent ici la zone 2.
- Phase n°3 (partie B de la figure) : élution de l'extrait. L'éluant (EL) et injecté sur la première cellule pour éluer l'extrait (EX), qui est collecté en volume sensiblement identique au bas de la première cellule. La cellule n°1 constitue ici la zone 1.
- Les phases n°2 et 3 sont de préférence opérées simultanément pour accroître la productivité du système.
- Phase n°4 : élution du raffinat. L'éluant (EL) est injecté en tête de la première cellule, et le raffinat (R) est collecté en volume sensiblement identique sortie de la cinquième. La cellule n°6 est ici une cellule tampon permettant d'assurer la séparation entre la queue de l'extrait et la tête du raffinat. Elle constitue la zone 4. Cette zone peut être omise dans le cas où le degré de pureté et/ou le rendement recherché est relativement limité.

Ces phases sont opérées dans l'ordre dans un mode de réalisation préféré, de 1 à 4. Leur enchaînement constitue une séquence complète.

Chaque séquence (phases n°1 à 4) est répétée six fois en décalant les entrées et sorties de cellules par incrémentation du numéro de cellule, de la gauche vers la droite du système : la charge est ainsi injectée en haut de cellule n°1 en séquence n°1, puis en haut de cellule n°2 en séquence n°2, etc.

Un cycle de production complet est réalisé après achèvement des six séquences successives, quand le point d'injection de la charge, initialement en entrée de cellule n°1, revient de nouveau en entrée de cellule n°1.

Dans ce qui précède, on a donné une description du système SSMB en référence au cas où les cellules correspondent à des colonnes. Ceci n'est pas limitatif, et l'invention s'applique aussi aux systèmes dans lesquels les cellules, ou encore compartiments, sont des parties de colonne.

Par ailleurs, le nombre de colonnes présentes en zones 1, 2, 3 et 4 peut varier en fonction de la qualité de séparation désirée. On peut donc concevoir des systèmes du même type avec une cellule, deux cellules, trois cellules, quatre cellules et jusqu'à douze cellules ou plus.

Le procédé peut encore être mis en œuvre dans une installation multi-colonnes non continue à boucle fermée ou à boucle ouverte, telle que le système DCC décrit dans le brevet WO 2007/012750.

Selon un mode de réalisation, le rendement de la séparation chromatographique est supérieur ou égal à 97%, de préférence supérieure ou égal à 98 %, de préférence supérieur ou égal à 99 %, de préférence supérieur ou égal à 99,5 %. Ce rendement correspond au pourcentage molaire de glycol du flux en entrée qui est récupéré dans la fraction enrichie en glycol.

Avantageusement, le taux d'élimination des sels dans la séparation chromatographique est supérieur ou égal à 50 %, de préférence à 80%, encore de préférence à 85 %, plus particulièrement supérieur ou égal à 90 % voire à 95 %. Ce taux d'élimination correspond au pourcentage molaire de sels dissous dans le flux en entrée qui ne se retrouve pas dans la fraction enrichie en glycol.

En particulier, le taux d'élimination du cation Na⁺ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination du cation K⁺ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination du cation Ca²⁺ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination du cation Mg²⁺ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination de l'anion Cl⁻ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination de l'anion SO₄²⁻ dans la séparation chromatographique est de préférence supérieur ou égal à 50 %, ou à 80 %, ou à 85 %, ou à 90 %, ou à 92 %, ou à 94 %, ou à 95 %.

De même, le taux d'élimination de l'anion CO₃²⁻ dans la séparation chromatographique est de préférence supérieur ou égal à 50 %, ou à 80 %, ou à 85 %, ou à 90 %, ou à 92 %, ou à 94 %, ou à 95 %.

L'invention permet avantageusement de se passer de toute étape de distillation du glycol.

De même, avantageusement, aucune autre étape de séparation des sels ou ions n'est effectuée avant ou après l'étape de séparation chromatographique. En particulier aucune étape de cristallisation et de décantation de cristaux n'est avantageusement prévue.

La fraction enrichie en glycol fait avantageusement l'objet d'un traitement complémentaire visant à la concentrer, c'est-à-dire à réduire sa teneur en eau. On peut utiliser ainsi une étape d'évaporation, ou une étape de séparation par osmose inverse.

De préférence, l'eau excédentaire collectée lors de ce traitement complémentaire est recyclée en tant qu'éluant dans l'étape chromatographique décrite ci-dessus.

La fraction enrichie en glycol récupérée grâce au procédé de l'invention (éventuellement après le traitement complémentaire mentionné ci-dessus) fournit un agent anti-hydrate régénéré, qui peut être utilisé dans l'extraction ou le transport d'hydrocarbures, afin d'assurer la stabilité du mélange polyphasique issu du gisement vis-à-vis des risques de formation d'hydrates de gaz.

Il faut noter qu'un avantage de l'invention est de ne nécessiter aucune (ou pratiquement aucune) régénération de la phase stationnaire, du fait du fonctionnement en mode d'exclusion d'ions et non en mode d'échange d'ions, et grâce au fait que la séparation n'est pas matériellement affectée par une évolution de la proportion des différents ions sur la résine au cours du temps. Par « *régénération de la phase stationnaire* » on entend le lavage de la phase stationnaire avec une solution de lavage spécifique destinée à reconditionner celle-ci dans une forme ionique particulière propre à permettre une séparation chromatographique efficace.

Le passage d'une solution de lavage dans la phase stationnaire peut toutefois dans certains cas être approprié pour nettoyer celle-ci en cas d'accumulation de contaminants tel que des espèces précipitées ou des composés organiques adsorbés.

Par exemple, dans le cadre d'une installation multi-colonnes (notamment du type SMB ou analogue), on peut prévoir une ou plusieurs zones de nettoyage, permettant de déconnecter une colonne de la boucle de séparation, afin de désorber périodiquement ou occasionnellement des contaminants, et le cas échéant de rééquilibrer la phase stationnaire avec le flux en entrée.

Selon un mode de réalisation, la phase stationnaire subit un lavage moins d'une fois par semaine, ou moins d'une fois toutes les deux semaines, ou moins d'une fois par mois, ou moins d'une fois par trimestre, ou moins d'une fois par semestre, ou moins d'une fois par an.

Selon un mode de réalisation, au moins 20 kg de glycol, de préférence au moins 40 kg de glycol, plus particulièrement au moins 90 kg de glycol, de manière préférée au moins 260 kg de glycol, de manière plus particulièrement préférée au moins 950 kg de glycol par litre de phase stationnaire passe dans l'unité chromatographique avant que la phase stationnaire subisse un lavage. Et, idéalement, la phase stationnaire ne subit aucun lavage, c'est-à-dire que celle-ci est uniquement soumise à l'injection du flux en entrée et d'éluant.

### EXEMPLES

Les exemples suivants illustrent l'invention sans la limiter.

### Exemple 1

Dans cet exemple, on injecte trois « *puises* » (charges de solutions à séparer de faible volume) différents, dans des conditions identiques, dans une unité chromatographique fonctionnant en mode d'exclusion d'ions. Ces trois expériences permettent de comparer la séparation entre des sels et du MEG, du DEG et du TEG respectivement.

Les trois solutions utilisées pour les trois pulses ont les compositions suivantes :
- 850 g/L de MEG + 13,2 g/L de NaCl + 15,5 g/L de CaCl₂, soit un rapport (cations divalents / cations totaux) exprimé en équivalents égal à 55 %.
- 850 g/L de DEG + 13,2 g/L de NaCl + 15,5 g/L de CaCl₂ soit un rapport (cations divalents / cations totaux) exprimé en équivalents égal à 55 %.
- 850 g/L de TEG + 13,2 g/L de NaCl + 15,5 g/L de CaCl₂ soit un rapport (cations divalents/ cations totaux) exprimé en équivalents égal à 55%.

Chaque pulse représente un volume de solution égal à 1 % du volume de résine (0,01 BV).

Chaque pulse est injecté en haut d'une colonne de chromatographie à double paroi, contenant une résine cationique forte sous forme divalente à 60 % environ (soit environ 40 % sous forme monovalente), suite à une mise en équilibre préalable avec un mélange d'origine industrielle contenant 34 % de cations divalents (par rapport à la quantité totale de cations, en équivalents). La colonne mesure 2,5 cm de diamètre, la hauteur de résine est de 90 cm, le volume de résine est de 440 mL et la température est fixée à 65°C.

Ensuite, on procède à une élution avec de l'eau déminéralisée, à une température de 65°C et à un débit de 20 mL/min. Le liquide sortant de la colonne est collecté et fractionné par un échantillonneur pour analyser la conductivité et la teneur en matières sèches (en degrés Brix) de chacune des fractions collectées. Lorsque toute la charge injectée a été éluée et la colonne rincée, un nouveau pulse est réalisé : les trois expériences sont ainsi réalisées en série.

La valeur de conductivité de chaque échantillon est représentative de la concentration en sels dans l'échantillon.

La teneur en matière sèches en degrés Brix (ou grammes de matière sèche pour 100 g de solution) est obtenue par une mesure d'indice de réfraction dans des solutions diluées (du fait que la teneur en matières sèches est proportionnelle à l'indice de réfraction).

La résine utilisée est une résine Applexion ® XA EG 1.

Les résultats obtenus sont représentés sur la **figure 3****.**

On constate que les sels et les glycols sont séparés de manière très efficace, avec un retour à la ligne de base entre le pic de sel et le pic de glycol. En outre, le temps de rétention de chaque glycol est le même bien que les masses molaires des trois glycols soient différentes (volumes de rétention de 0,88 BV pour le MEG, 0,92 BV pour le DEG et 0,90 BV pour le TEG, soit en moyenne 0,90 BV).

De même, le temps de rétention des sels ne varie pas d'un pulse à l'autre (volume de rétention quasi-constant à 0,47 BV).

Les performances de séparation sont donc sensiblement identiques pour le MEG, le DEG et le TEG.

### Exemple 2

On répète la méthodologie de l'exemple 1, en étudiant la séparation d'un pulse d'une solution industrielle de glycol riche, provenant d'une plateforme off-shore.

Cette solution est composée de 840 g/L de MEG et de 29 g/L de sels, avec un rapport en équivalents (cations divalents / cations totaux) égal à 34 %. Les conditions de débit, température et volume injecté sont les mêmes que précédemment.

La colonne chromatographique est remplie avec une résine cationique forte qui est conditionnée sous trois formes différentes :
- 100 % potassium ;
- 100 % calcium ;
- 60 % divalente (Ca majoritaire) / 40 % monovalente (Na majoritaire), c'est-à-dire une forme équilibrée obtenue après saturation de la résine avec la solution industrielle.

La résine utilisée est une résine Applexion ® XA EG 1.

Les résultats obtenus sont représentés sur la **figure 4****.**

Ces résultats montrent :
- une séparation complète entre le pic de sel et le pic de MEG quelle que soit la forme ionique de la résine ;
- une augmentation de seulement 10 à 15 % des temps de rétention à la fois du sel et du MEG entre les formes extrêmes de la résine, soit la forme K et la forme Ca. En pratique, la résine évolue entre sa forme initiale (typiquement K) et une forme ou les ions K et Ca sont à l'équilibre sur la résine, et donc les variations des temps de rétention sont d'autant plus faibles.

Cet exemple démontre que l'efficacité de l'exclusion des ions en solution, et donc de la séparation entre le glycol et les sels de sodium, potassium, calcium et magnésium en solution, n'est pas fonction de la forme ionique de la résine cationique forte, à condition que les phases soient à l'état d'équilibre (échanges d'ions entre ions monovalents et divalents de la résine si le rapport monovalent / divalent du mélange à séparer varie au cours des injections).

### Exemple 3

Dans cet exemple, on s'intéresse à un profil de surcharge en produit de la résine réalisé sur la colonne de chromatographie à partir d'un produit à très forte teneur en sels.

Le mélange traité utilisé comprend 225 g/L de MEG et 247 g/L de sels, avec un rapport cations divalent / cations totaux de 34 %, identique à celui utilisé dans les exemples précédents.

Une surcharge consiste à saturer la résine avec un volume important de produit industriel représentatif afin d'étudier les effets thermodynamiques et compétitifs des différents composés.

Ainsi, un volume de 1,5 BV de mélange est percolé à travers la colonne à 65°C, au débit de 20 mL/min ; le liquide sortant est collecté et fractionné en une vingtaine d'échantillons instantanés. Lorsque ce volume de mélange est passé, la colonne est rincée avec 2 BV d'eau déminéralisée. Durant la phase de rinçage, un échantillonnage est effectué de la même façon que pour la phase de saturation en produit.

Les échantillons collectés sont ensuite analysés pour mesurer les concentrations en MEG, Na, Ca, Cl, K et Mg.

La résine utilisée est une résine Applexion ® XA EG 2.

Les résultats obtenus sont visibles sur la **figure 5****.**

Les profils des espèces ioniques montrent que les différents sels minéraux contenus dans le mélange traité ont la même affinité pour la résine : ils sortent en même temps de la colonne et sont également rincés en même temps. Comme pour les pulses des exemples précédents, on observe que les sels sortent en premier de la résine et que le MEG sort environ 0,3 BV plus tard.

Cette séparation entre les espèces à l'adsorption et à la désorption sur une résine équilibrée avec le mélange à traiter démontre la robustesse de l'invention, qui est remarquable au vu de la forte concentration en sels appliquée de 250 g/L.

### Exemple 4

Dans cet exemple, on s'intéresse au profil de matière en MEG et en sels observé dans un système chromatographique continu de type SSMB à 6 colonnes du type de la **figure 2**, opéré à une température de 60°C.

La résine utilisée est la résine Applexion ® XA EG 1.

Le mélange à séparer est le même que celui de l'exemple 2. Il est injecté de façon discontinue entre les points de collecte de l'extrait et du raffinat. Le MEG qui est retenu par la résine se concentre dans l'extrait alors que les sels qui sont exclus sont élués à l'opposé vers la zone de raffinat. Les deux fractions, extrait et raffinat, sont soutirées de manière semi-continue du système de la même manière que l'injection du mélange à séparer et de l'eau.

Les résultats obtenus sont visibles sur la **figure 6****.**

Ce profil correspond à un taux de récupération de 99,8 % du MEG dans la fraction extrait (quantité de MEG récupérée dans l'extrait par rapport à la quantité totale injectée dans le système) et un taux d'élimination des sels de 95,5 % dans la fraction raffinat (quantité de sels éliminée dans le raffinat par rapport à la quantité totale de sels injectée dans le système).

## Revendications

1. Procédé de purification comprenant :
- la fourniture d'un flux comprenant un glycol, des ions monovalents et des ions multivalents ;
- le traitement de ce flux par chromatographie d'exclusion d'ions comprenant :
▪ l'injection du flux dans une unité chromatographique comprenant une phase stationnaire échangeuse d'ions ;
▪ l'injection d'un éluant dans l'unité chromatographique ;
▪ la collecte d'une fraction en sortie de l'unité chromatographique ;
- optionnellement, une étape ultérieure de concentration de la fraction collectée ;
la fraction collectée étant enrichie en glycol et appauvrie en ions monovalents et en ions multivalents par rapport au flux.

2. Procédé selon la revendication 1, dans lequel le glycol est choisi parmi le monoéthylène glycol, le diéthylène glycol, le triéthylène glycol et les mélanges de ceux-ci ; et/ou dans lequel l'éluant est die l'eau, de préférence de l'eau déminéralisée ou de l'eau issue de l'étape de concentration de la fraction collectée.

3. Procédé selon l'une des revendications 1 à 2, dans lequel les ions monovalents comprennent des cations monovalents, et les ions multivallents comprennent des cations divalents ; et dans lequel, de préférence, les cations monovalents comprennent des cations Na⁺ et/ou K+, et les cations divalents comprennent des cations Ca²⁺ et/ou Mg²⁺ ; et/ou dans lequel, de préférence, le flux présente un rapport en équivalents de cations divalents sur les cations totaux de 5 à 90 %. préférablement de 8 à 75 % et de manière préférée de 10 à 50 %.

4. Procédé selon l'une des revendications 1 à 3, dans lequel l'unité chromatographique est une unité multi-colonnes à lit non-statique, et de préférence une unité chromatographique à lit mobile simulé, ou à lit mobile réel, ou à lit mobile simulé amélioré, ou à lit mobile simulé séquentiel, et de manière particulièrement préférée à lit mobile simulé séquentiel.

5. Procédé selon l'une des revendications 1 à 4, dans lequel la phase stationnaire est une résine cationique qui est en partie sous forme cationique monovalente et en partie sous forme cationique divalente, la forme cationique monovalente et la forme cationique divalente correspondant de préférence à des ions présents dans le flux.

6. Procédé selon l'une des revendications 1 à 5, dans lequel, dans la fraction collectée. au moins 50 mol.%, de préférence au moins 80 mol.%, encore de préférence au moins 85 mol.%, de manière plus particulièrement préférée au moins 90 mol.% et de manière encore plus préférée au moins 95 mol.% des ions monovalents et des ions multivalents présents dans le flux sont éliminés.

7. Procédé selon l'une des revendications 1 à 6, dans lequel au moins 20 kg de glycol, de préférence au moins 40 kg de glycol, de manière plus particulièrement préférée au moins 90 kg de glycol par litre de phase stationnaire passe dans l'unité chromatographique avant que la phase stationnaire subisse un lavage ; et, idéalement, la phase stationnaire ne subit aucun lavage.

8. Procédé de régénération d'un agent anti-hydrate, comprenant :
- la fourniture d'un mélange multiphasique comprenant des hydrocarbures, un glycol et des sels dissous ;
- la séparation des hydrocarbures de ce mélange, permettant de récupérer une phase aqueuse ;
- l'application du procédé de purification de l'une des revendications 1 à 7 à la phase aqueuse en tant que flux, et la récupération d'une composition d'agent anti-hydrate régénérée sous la forme de la fraction collectée, et le cas échéant concentrée ;
ledit procédé comprenant option nettement une étape de clarification de la phase aqueuse avant l'application du procédé de purification, de préférence par filtration et/ou une étape de concentration de la phase aqueuse.

9. Installation de purification comprenant :
- une ligne d'amenée comprenant un flux comprenant un glycol, des ions monovalents et des ions multivalents ;
- une ligne d'amenée comprenant un éluant ;
- une unité chromatographique comprenant une phase stationnaire échangeuse d'ions, alimentée par la ligne d'amenée comprenant le flux et par la ligne d'amenée comprenant l'éluant et configurée pour réaliser une chromatographie d'exclusion d'ions, et dans laquelle la phase stationnaire est une résine cationique qui est en partie sous forme cationique monovalente et en partie sous forme cationique divalente, la forme cationique monovalente et la forme cationique divalente correspondant à des ions présents dans le flux ;
- une ligne de collecte comprenant une fraction enrichie en glycol et appauvrie en ions monovalents et en ions multivalents par rapport au flux, connectée en sortie de l'unité chromatographique, et alimentant de préférence une unité de concentration de la fraction enrichie en glycol et appauvrie en ions monovalents et en ions multivalents.

10. Installation selon la revendication 9, dans laquelle le glycol est choisi parmi le monoéthylène glycol, le diéthylène glycol, le triéthylène glycol et les combinaisons de ceux-ci.

11. Installation selon l'une des revendications 9 à 10, dans laquelle les ions monovalents comprennent des cations monovalents, et les ions multivalents comprennent des cations divalents ; et de préférence, les cations monovalents comprennent des cations Na⁺ et/ou K+, et les cations divalents comprennent des cations Ca²⁺ et/ou Mg²⁺ ; et/ou, de préférence, le flux présente un rapport en équivalents de cations divalents sur les cations totaux de 5 à 90 %, ou de 8 à 75 %, ou de 10 à 50 %.

12. Installation selon l'une des revendications 9 à 11, dans laquelle l'unité chromatographique est une unité multi-colonnes à lit non-statique, et de préférence une unité chromatographique à lit mobile simulé, ou à lit mobile réel, ou à lit mobile simulé amélioré, ou à lit mobile simulé séquentiel, et de manière particulièrement préférée à lit mobile simulé séquentiel.

13. Installation selon l'une des revendications 9 à 12, dans lequel l'éluant est de l'eau, de préférence de l'eau déminéralisée ; et dans laquelle, de manière particulièrement préférée, une ligne de recyclage d'eau est prévue entre l'unité de concentration de la fraction enrichie en glycol et appauvrie en ions monovalents et en ions multivalents et la ligne d'amenée comprenant l'éluant.

14. Installation de régénération d'un agent-hydrate, comprenant ;
- une ligne d'amenée comprenant un mélange multiphasique comprenant des hydrocarbures, un glycol et des sels dissous ;
- une unité de séparation des hydrocarbures alimentée par cette conduite d'amenée comprenant le mélange polyphasique ;
- une ligne de prélèvement comprenant une phase aqueuse issue de l'unité de séparation des hydrocarbures ;
- l'installation de purification selon l'une des revendications 9 à 14, dans laquelle la ligne d'amenée comprenant le flux est alimentée par la ligne de prélèvement comprenant la phase aqueuse ;
- une ligne de collecte comprenant une composition d'agent anti-hydrate régénérée, alimentée par la ligne de collecte comprenant la fraction enrichie en glycol et appauvrie en ions monovalents et en ions multivalents ou le cas échéant par une ligne de collecte issue de l'unité de concentration de la fraction enrichie en glycol et appauvrie en ions monovalents et en ions multivalents ;
- optionnellement, une unité de clarification de la phase aqueuse, de préférence une unité de filtration, et/ou une unité de concentration de la phase aqueuse, en amont de l'installation de purification.

## Patentansprüche

1. Reinigungsverfahren, umfassend:
- das Bereitstellen eines Stroms, umfassend ein Glycol, einwertige Ionen und mehrwertige Ionen;
- das Bearbeiten dieses Stroms durch Ionenausschlusschromatographie, umfassend:
▪ das Einleiten des Stroms in eine Chromatographieeinheit, umfassend eine stationäre Ionenaustauschphase;
▪ das Einleiten eines Elutionsmittels in die Chromatographieeinheit;
▪ das Sammeln einer Fraktion am Ausgang der Chromatographieeinheit;
- optional einen späteren Konzentrationsschritt der gesammelten Fraktion;
wobei die gesammelte Fraktion mit Glycol angereichert und von einwertigen Ionen und von mehrwertigen Ionen abgereichert in Bezug auf den Strom ist.

2. Verfahren nach Anspruch 1, wobei das Glycol ausgewählt ist aus Monoethylenglycol, Diethylenglycol, Triethylenglycol und den Gemischen derselben; und/oder wobei das Elutionsmittel Wasser ist, vorzugsweise entmineralisiertes Wasser oder Wasser aus dem Konzentrationsschritt der gesammelten Fraktion.

3. Verfahren nach einem der Ansprüche 1 bis 2, wobei die einwertigen Ionen einwertige Kationen umfassen und die mehrwertigen Ionen zweiwertige Kationen umfassen; und wobei vorzugsweise die einwertigen Kationen Na⁺- und/oder K⁺-Kationen umfassen und die zweiwertigen Kationen Ca²⁺- und/oder Mg²⁺-Kationen umfassen; und/oder wobei vorzugsweise der Strom ein Verhältnis zweiwertige Kationenäquivalente zu Kationen insgesamt von 5 bis 90 %, bevorzugt von 8 bis 75 % und in bevorzugter Weise von 10 bis 50 % aufweist.

4. Verfahren nach einem der Ansprüche 1 bis 3, wobei die Chromatographieeinheit eine Mehrkolonneneinheit mit nichtstatischem Bett und vorzugsweise eine Chromatographieeinheit mit simuliertem beweglichem Bett oder mit tatsächlich mobilem Bett oder mit verbessertem simuliertem beweglichem Bett oder mit sequentiell simuliertem beweglichem Bett und in besonders bevorzugter Weise mit sequentiell simuliertem beweglichem Bett ist.

5. Verfahren nach einem der Ansprüche 1 bis 4, wobei die stationäre Phase ein kationisches Harz ist, das teilweise in einwertiger kationischer Form und teilweise in zweiwertiger kationischer Form vorliegt, wobei die einwertige kationische Form und die zweiwertige kationische Form vorzugsweise Ionen entsprechen, die in dem Strom vorhanden sind.

6. Verfahren nach einem der Ansprüche 1 bis 5, wobei in der gesammelten Fraktion mindestens 50 mol.%, vorzugsweise mindestens 80 mol.%, noch vorzugsweiser mindestens 85 mol.%, in besonders bevorzugter Weise mindestens 90 mol.% und in noch bevorzugterer Weise mindestens 95 mol.% der in dem Strom vorhandenen einwertigen Ionen und der mehrwertigen Ionen entfernt werden.

7. Verfahren nach einem der Ansprüche 1 bis 6, wobei mindestens 20 kg Glycol, vorzugsweise mindestens 40 kg Glycol, in besonders bevorzugter Weise mindestens 90 kg Glycol je Liter stationäre Phase durch die Chromatographieeinheit gelangt, bevor die stationäre Phase gewaschen wird; und in idealer Weise die stationäre Phase überhaupt nicht gewaschen wird.

8. Regenerationsverfahren eines Anithydratmittels, umfassend:
- das Bereitstellen eines Mehrphasengemischs, umfassend Kohlenwasserstoffe, ein Glycol und gelöste Salze;
- das Trennen der Kohlenwasserstoffe von diesem Gemisch, was erlaubt, eine wässrige Phase zurückzugewinnen;
- das Anwenden des Reinigungsverfahrens von einem der Ansprüche 1 bis 7 auf die wässrige Phase als Strom und das Zurückgewinnen einer Zusammensetzung eines regenerierten Antihydratmittels in Form der gesammelten Fraktion und gegebenenfalls konzentriert;
wobei das Verfahren optional einen Klärschritt der wässrigen Phase vor der Anwendung des Reinigungsverfahrens, vorzugsweise durch Filtration, und/oder einen Konzentrationsschritt der wässrigen Phase umfasst.

9. Reinigungsanlage, umfassend:
- eine Zufuhrleitung, umfassend einen Strom, umfassend ein Glycol, einwertige Ionen und mehrwertige Ionen;
- eine Zufuhrleitung, umfassend ein Elutionsmittel;
- eine Chromatographieeinheit, umfassend eine stationäre Ionenaustauschphase, die von der Zufuhrleitung, die den Strom umfasst, und von der Zufuhrleitung, die das Elutionsmittel umfasst, versorgt wird und ausgelegt ist, um eine Ionenausschlusschromatographie durchzuführen, und in welcher die stationäre Phase ein kationisches Harz ist, das teilweise in einwertiger kationischer Form und teilweise in zweiwertiger kationischer Form vorliegt, wobei die einwertige kationische Form und die zweiwertige kationische Form Ionen entsprechen, die in dem Strom vorhanden sind;
- eine Sammelleitung, umfassend eine mit Glycol angereicherte und von einwertigen Ionen und von mehrwertigen Ionen in Bezug auf den Strom abgereicherte Fraktion, die am Ausgang der Chromatographieeinheit angeschlossen ist und vorzugsweise eine Konzentrationseinheit der mit Glycol angereicherten und von einwertigen Ionen und von mehrwertigen Ionen abgereicherten Fraktion versorgt.

10. Anlage nach Anspruch 9, wobei das Glycol ausgewählt ist aus Monoethylenglycol, Diethylenglycol, Triethylenglycol und den Gemischen derselben.

11. Anlage nach einem der Ansprüche 9 bis 10, wobei die einwertigen Ionen einwertige Kationen umfassen und die mehrwertigen Ionen zweiwertige Kationen umfassen; und vorzugsweise die einwertigen Kationen Na⁺- und/oder K⁺-Kationen umfassen und die zweiwertigen Kationen Ca²⁺- und/oder Mg²⁺-Kationen umfassen; und/oder vorzugsweise der Strom vorzugsweise ein Verhältnis zweiwertige Kationenäquivalente zu Kationen insgesamt von 5 bis 90 % oder von 8 bis 75 % oder von 10 bis 50 % aufweist.

12. Anlage nach einem der Ansprüche 9 bis 11, wobei die Chromatographieeinheit eine Mehrkolonneneinheit mit nichtstatischem Bett und vorzugsweise eine Chromatographieeinheit mi simuliertem beweglichem Bett oder mit tatsächlich mobilem Bett oder mit verbessertem simuliertem beweglichem Bett oder mit sequentiell simuliertem beweglichem Bett und in besonders bevorzugter Weise mit sequentiell simuliertem beweglichem Bett ist.

13. Anlage nach einem der Ansprüche 9 bis 12, wobei das Elutionsmittel Wasser ist, vorzugsweise entmineralisiertes Wasser; und wobei in besonders bevorzugter Weise eine Wasserrecyclingleitung zwischen der Konzentrationseinheit der mit Glycol angereicherten und von einwertigen Ionen und von mehrwertigen Ionen abgereicherten Fraktion und der Zufuhrleitung, die das Elutionsmittel umfasst, vorgesehen ist.

14. Regenerationsanlage eines Hydratmittels, umfassend:
- eine Zufuhrleitung, umfassend ein Mehrphasengemisch, umfassend Kohlenwasserstoffe, ein Glycol und gelöste Salze;
- eine Separationseinheit der Kohlenwasserstoffe, die durch diese Zufuhrleitung versorgt wird, die das Polyphasengemisch umfasst;
- eine Entnahmeleitung, umfassend eine wässrige Phase, die aus der Separationseinheit der Kohlenwasserstoffe hervorgegangen ist;
- die Reinigungsanlage nach einem der Ansprüche 9 bis 14, wobei die Zufuhrleitung, die den Strom umfasst, von der Entnahmeleitung versorgt wird, die die wässrige Phase umfasst;
- eine Sammelleitung, umfassend eine Zusammensetzung regenerierten Antihydratmittels, die von der Sammelleitung versorgt wird, die die mit Glycol angereicherte und von einwertigen Ionen und von mehrwertigen Ionen abgereicherte Fraktion umfasst und gegebenenfalls von einer Sammelleitung, die aus einer Konzentrationseinheit der mit Glycol angereicherten und von einwertigen Ionen und von mehrwertigen Ionen abgereicherten Fraktion hervorgegangen ist;
- optional eine Kläreinheit für die wässrigen Phase, vorzugsweise eine Filtrationseinheit und/oder eine Konzentrationseinheit für die wässrige Phase vor der Reinigungsanlage.

## Claims

1. A purification method comprising:
- providing a flow comprising a glycol, monovalent ions and multivalent ions;
- treating this flow with ion exclusion chromatography comprising:
▪ injecting the flow into a chromatographic unit comprising an ion exchange stationary phase;
▪ injecting an eluent into the chromatographic unit;
▪ collecting a fraction at the outlet of the chromatographic unit;
- optionally, a subsequent step of concentrating the collected fraction; the collected fraction being enriched with glycol and depleted of monovalent ions and multivalent ions relative to the flow.

2. The method according to claim 1, wherein the glycol is selected from monoethylene glycol, diethylene glycol, triethylene glycol and mixtures thereof; and/or wherein the eluent is water, preferably demineralized water or water derived from the step of concentrating the collected fraction.

3. The method according to one of claims 1 to 2, wherein the monovalent ions comprise monovalent cations, and the multivalent ions comprise divalent cations; and wherein preferably the monovalent cations comprise Na⁺ and/or K⁺ cations, and the divalent cations comprise Ca²⁺ and/or Mg²⁺ cations; and/or wherein preferably the flow has a ratio, in equivalents, of divalent cations to total cations of 5 to 90 %, preferably 8 to 75 % and more preferably 10 to 50 %.

4. The method according to one of claims 1 to 3, wherein the chromatographic unit is a multi-column unit with non-static bed, and preferably a chromatographic unit with simulated moving bed, or actual moving bed, or improved simulated moving bed, or sequential simulated moving bed, and more preferably sequential simulated moving bed.

5. The method according to one of claims 1 to 4, wherein the stationary phase is a cationic resin which is partly in monovalent cationic form and partly in divalent cationic form, the monovalent cationic form and the divalent cationic form preferably corresponding to ions present in the flow.

6. The method according to one of claims 1 to 5, wherein, in the collected fraction, at least 50 mol.%, preferably at least 80 mol.%, more preferably at least 85 mol.%, further preferably at least 90 mol.% and still further preferably at least 95 mol.% of the monovalent ions and multivalent ions present in the flow are removed.

7. The method according to one of claims 1 to 6, wherein at least 20 kg of glycol, preferably at least 40 kg of glycol, more preferably at least 90 kg of glycol per liter of stationary phase passes through the chromatographic unit before the stationary phase is subjected to washing; and ideally the stationary phase is not subjected to any washing.

8. A method to regenerate an anti-hydrate agent, comprising:
- providing a multiphase mixture comprising hydrocarbons, a glycol and dissolved salts;
- separating the hydrocarbons from this mixture, so as to recover an aqueous phase;
- applying the purification method of one of claims 1 to 7 to the aqueous phase as a flow, and recovering a regenerated composition of anti-hydrate agent in the form of the collected, and if appropriate concentrated, fraction;
said method comprising optionally a step of clarifying the aqueous phase before applying the purification method, preferably by filtration, and/or a step of concentrating the aqueous phase.

9. A purification installation comprising:
- a feed line comprising a flow comprising a glycol, monovalent ions and multivalent ions;
- a feed line comprising an eluent;
- a chromatographic unit comprising an ion exchange stationary phase, supplied by the feed line comprising the flow and the feed line comprising the eluent and configured to perform ion exclusion chromatography, and wherein the stationary phase is a cationic resin which is partly in monovalent cationic form and partly in divalent cationic form, the monovalent cationic form and the divalent cationic form corresponding to ions present in the flow;
- a collecting line comprising a fraction enriched with glycol and depleted of monovalent ions and multivalent ions relative to the flow, connected to the outlet of the chromatographic unit, and that preferably feeds a unit for concentrating the fraction enriched with glycol and depleted of monovalent ions and multivalent ions.

10. The installation according to claim 9 wherein the glycol is selected from monoethylene glycol, diethylene glycol, triethylene glycol and combinations thereof.

11. The installation according to one of claims 9 to 10, wherein the monovalent ions comprise monovalent cations, and the multivalent ions comprise divalent cations; and preferably the monovalent cations comprise Na⁺ and/or K⁺ cations, and the divalent cations comprise Ca²⁺ and/or Mg²⁺ cations; and/or, preferably, the flow has a ratio, in equivalents, of divalent cations to total cations of 5 to 90%, or 8 to 75%, or 10 to 50%.

12. The installation according to one of claims 9 to 11, wherein the chromatographic unit is a multi-column unit with non-static bed, and preferably a chromatographic unit with simulated moving bed, or actual moving bed, or improved simulated moving bed, or sequential simulated moving bed, and more preferably sequential simulated moving bed.

13. The installation according to one of claims 9 to 12, wherein the eluent is water, preferably demineralized water; and wherein, in particularly preferred manner, a water recycling line is provided between the unit for concentrating the fraction enriched with glycol and depleted of monovalent ions and multivalent ions and the feed line comprising the eluent.

14. An installation to regenerate an anti-hydrate agent, comprising:
- a feed line comprising a multiphase mixture comprising hydrocarbons, a glycol and dissolved salts;
- a hydrocarbon separation unit supplied by this feed line comprising the multiphase mixture;
- a draw-off line comprising an aqueous phase from the hydrocarbon separation unit;
- the purification installation according to one of claims 9 to 14, wherein the feed line comprising the flow is supplied by the draw-off line comprising the aqueous phase;
- a collecting line comprising a regenerated composition of anti-hydrate agent, supplied by the collecting line comprising the fraction enriched with glycol and depleted of monovalent ions and multivalent ions, or, if appropriate, by a collecting line from the unit for concentrating the fraction enriched with glycol and depleted of monovalent ions and multivalent ions;
- optionally, a unit for clarifying the aqueous phase, preferably a filtration unit, and/or a unit for concentrating the aqueous phase, upstream of the purification installation.
